# EUROPÄISCHE PATENTSCHRIFT

(11) **EP 3 155 429 B1**
(45) Veröffentlichungstag und Bekanntmachung des Hinweises auf die Patenterteilung: **10.04.2019**
(21) Anmeldenummer: 15745377.0
(22) Anmeldetag: 12.06.2015
(51) Int. Cl.: G01N 33/543

(54) **SPEZIFISCHE PROTEINMARKIERUNG SOWIE VERFAHREN ZUR IDENTIFIZIERUNG DER STATISTISCHEN VERTEILUNG DER PROTEINSTÖCHIOMETRIE**
SPECIFIC PROTEIN MARKER AND METHOD FOR IDENTIFYING THE STATISTIC DISTRIBUTION OF PROTEIN STOICHIOMETRY
MARQUAGE SPÉCIFIQUE DE PROTÉINES ET PROCÉDÉS D'IDENTIFICATION DE LA RÉPARTITION STATISTIQUE DE LA STOECHIOMÉTRIE DES PROTÉINES

(30) Priorität: 13.06.2014 DE 102014108331
(43) Veröffentlichungstag der Anmeldung: 19.04.2017
(73) Patentinhaber: Leibniz-Institut für Neue Materialien gemeinnützige GmbH, 66123 Saarbrücken (DE)
(72) Erfinder: DE JONGE, Niels, 66386 St. Ingbert (DE); PECKYS, Diana, B., 66386 St. Ingbert (DE)
(74) Vertreter: Patentanwälte Gierlich & Pischitzis Partnerschaft mbB
(86) Internationale Anmeldenummer: PCT/DE2015/100238
(87) Internationale Veröffentlichungsnummer: WO 2015/188814

(56) Entgegenhaltungen:
- WO-A1-2014/016465
- WO-A2-2004/020453
- US-A1- 2007 249 064
- KYOUNG LEE ET AL: "Rapid Detection of Intracellular Nanoparticles by Electron Microscopy", JOURNAL OF ANALYTICAL SCIENCE & TECHNOLOGY, Bd. 1, Nr. 1, 1. Januar 2010 (2010-01-01), Seiten 71-73, XP002746165, DOI: 10.5355/JAST.2010.71
- WATANABE SHIGEKI ET AL: "Protein localization in electron micrographs using fluorescence nanoscopy", NATURE METHODS, Bd. 8, Nr. 1, Januar 2011 (2011-01), Seite 80, XP055220517,

## Beschreibung

Die vorliegende Erfindung betrifft eine spezifische Proteinmarkierung sowie Verfahren zur Identifizierung der statistischen Verteilung der Eiweißkomplexstöchiometrie, auch Proteinstöchiometrie genannt.

Zelluläre Funktionen werden durch die molekularen Mechanismen aus Proteinen, DNA, Peptiden usw. geregelt, welche dynamisch zusammengesetzt werden zu funktionellen makromolekularen Komplexen. Im Laufe des letzten halben Jahrhunderts wurden zahlreiche technische Fortschritte in der biomedizinischen Forschung und wichtige Einblicke in die komplexen molekularen Wechselwirkungen erzielt. Dennoch sind viele wissenschaftliche Fragen über das Zusammenspiel von makromolekularen Komplexen in Bezug auf die Zellfunktion nicht vollständig geklärt, unter anderem weil Verfahren fehlen, welche diese in intakten Zellen untersuchen können. Als Folge ergeben biochemische Studien manchmal widersprechende Ergebnisse. Des Weiteren können diese biochemischen Studien auch keine Informationen über den ursprünglichen Ort der makromolekularen Komplexe innerhalb der Zellen bereitstellen.

Des Weiteren ist das Abbilden von Zellkomponenten innerhalb von ganzen Zellen schwierig, da die Dimensionen im Nanobereich liegen.

Eine wichtige Frage ist, unter welchen Konditionen und an welchem Ort Dimerisierung entsteht. Dieses Beispiel gibt nur eine von vielen Fragen wieder, die mit dem Zusammenspiel von ungefähr 20.000 Proteinen in einer typischen eukaryotische Zelle zusammenhängen.

Ein Biologe oder ein Biophysiker würde idealerweise die Möglichkeit eines schnellen Mikroskops mit einer räumlichen Auflösung von wenigen Nanometern haben, um die Lokalisation von individuellen Subeinheiten in einem Proteinkomplex zu identifizieren. Idealerweise würde sich die Zelle in ihrer natürlichen wässrigen Umgebung befinden und die Zelle würde während der Lokalisation der Subeinheiten nicht beschädigt. Insbesondere Membranproteine, die die Hauptgruppe heutiger Wirkstoff-Targets darstellen, sind mit den zurzeit bekannten mikroskopischen Techniken schwierig zu untersuchen.

Die Nachteile der gängig verwendeten Lichtmikroskope werden im Folgenden diskutiert.

Zelluläre Funktionen werden oftmals mit Fluoreszenzmikroskopen untersucht, wobei Zellen verwendet werden, die fluoreszierende Proteinmarker aufweisen. Superauflösende-Fluoreszenzmikroskopie bietet Bilder mit einer räumlichen Auflösung von einigen zehn Nanometern. Jedoch ist die Auflösung durch die verschiedenen Eigenschaften der Fluorophore und, im Fall von stimulierter Emissions-Depletions Mikroskopie (STED), die Intensität des Lasersstrahls begrenzt, derart, dass ein Wert von ca. 30 nm für die praktischen Abbildungsbedingungen an ganzen Zellen erreicht wird. Diese Längenskala ist jedoch nicht ausreichend, um einzelne markierte Proteine in Komplexen widerzuspiegeln. Dies wird jedoch benötigt, um die Zellfunktion auf molekularer Ebene zu untersuchen. Betrachtet man zum Beispiel die Forschung an dem epidermalen Wachstumsfaktor (epidermal growth factor, EGF)-Membranrezeptor (EGFR), wird schnell ersichtlich, dass Techniken benötigt werden, um zu unterscheiden, ob ein Rezeptor in einer monomeren, dimeren oder einer Cluster-Konfiguration vorliegt.

Andere technologische Entwicklungen der Lichtmikroskopie, wie Plasmonresonanz-Kopplung, Totalreflexionsbeleuchtung (TIRFM) und kalibrierte Einzelphotonendetektion wurden verwendet, um EGFR und andere Membranrezeptoren zu untersuchen. Hierbei muss jedoch im Auge behalten werden, dass diese Methoden in ihrer räumlichen Auflösung beugungsbegrenzt sind und auf umfangreiche Bildverarbeitung und Berechnungen zurückgreifen müssen. Diese Methoden visualisieren nicht direkt die einzelnen EGFRs, sondern ermöglichen nur indirekte Rückschlüsse auf Ensembles von EGFRs.

Eine weitere Methode der Lichtmikroskopie, der sogenannte Förster-Resonanzenergietransfer (FRET), ist sehr empfindlich für kurze intermolekulare Abstände (<10 nm) und wurde erfolgreich für die Untersuchung von Konformationsänderungen von EGFRs angewendet. Aber auch FRET ist eine Ensemble-Mittelungstechnik und muss mit großer Vorsicht interpretiert werden, wenn es zu den kleinsten Clustern, den Dimeren, kommt. Dies beruht darauf, dass die FRET-Daten, welche Dimere indizieren, auch aus einem Gemisch von Monomeren und großen Clustern (mit - im Durchschnitt - zwei Molekülen in enger räumlicher Beziehung) entstehen können.

Ein weiterer Ansatz ist die Abschätzung der Anzahl von Proteinkomplex-Untereinheiten in präparierten Plasmamembran-Patches durch diskrete Bleichschritte der Fluoreszenzsignale unter Verwendung von zeitaufgelöster Mikroskopie. Jedoch erfordert diese Technik eine sehr geringe Dichte des fluoreszenz-markierten Proteinkomplexes innerhalb eines Bereiches von der Größe eines Beugungsflecks, benötigt stochastische Annäherungen, führt zu praktischen Schwierigkeiten bei der Untersuchung von intakten Zellen, ist auf insgesamt fünf Untereinheiten des gleichen Typs begrenzt und die Ergebnisse müssen durch biochemische Methoden oder Elektronenmikroskopie überprüft werden, um die Genauigkeit der Interpretation zu gewährleisten.

Die Methode der Wahl für nanoskalige Studien ist die Elektronenmikroskopie. Aktuelle nanoskalige Methoden, um Zellen zu untersuchen, verwenden zumeist Transmissionselektronenmikroskopie (TEM) von dünnen in Kunststoff eingebetteten Zellschnitten, Gefrierschnitten, Kanten von eingefrorenen Zellen oder Frakturen von Zellproben. Alternativ können die Zellen geschnitten und in einem seriellen Prozess abgebildet werden. In allen Fällen werden feste Proben für Untersuchungen im Vakuum des Elektronenmikroskops benötigt, wobei demnach die Zellen nicht in flüssigem Zustand verbleiben. Auch sind der Kontrast und die Auflösung in der Regel zu gering für eine direkte Identifizierung der einzelnen Proteine in eukaryotischen Zellen. Daher werden spezifische Immun-Markierungen aus schweren Materialien, z.B. Gold-Nanopartikel (Au-NP), benötigt, die an spezifische Antikörper gekoppelt sind, um Proteine in Dünnschnitten zu lokalisieren. Die Nanopartikel (NP) bieten ausreichend Kontrast für die Elektronenmikroskopie, so dass die Proteine in der dicht gepackten Zellmatrix nachgewiesen werden können.

Eine noch höhere Auflösung kann für die Bildgebung von markierten Proteinen, die in der biologischen Struktur eingebettet sind, durch Verwendung eines Rastertransmissionselektronenmikroskops (STEM) erwartet werden. Das STEM bietet einen starken Kontrast von Markierungen aus schweren Materialien aufgrund des Ordnungszahl-(Z)-Kontrasts. Dieses Prinzip wird verwendet, um Markierungen in ganzen Zellen, die sich in einer Flüssigkeitsschicht befinden, zu erkennen.

Obwohl es durch Elektronenmikroskopie ermöglicht wird, die Positionen von Proteinen mit ausreichender Präzision festzustellen, ist diese Art der Mikroskopie gegenwärtig durch die Verfügbarkeit spezifischer Markierungen beschränkt.

Daher verwenden viele Studien Immunogoldmarkierungen, wobei jedoch die Nanopartikelmarkierungen an den interessierenden Proteinen über ziemlich lange Linker (Verbinder) befestigt werden. Der Linker, z.B. bestehend aus ein oder zwei Antikörpern, ist flexibel und hat eine typische Länge von 30 nm. Als direkte Folge der Länge des Linkers kann die genaue Lage des Proteins nicht mit einer höheren Genauigkeit als 30 nm bestimmt werden. Dies schränkt die Anwendung stark ein.

Die Immunogoldmarkierung liefert Informationen über die Anwesenheit einer bestimmten Proteinspezies, zum Beispiel in einer Organelle, aber kann keine Informationen über den genauen Ort des Proteins mit einer besseren Genauigkeit als 30 nm zeigen. Es ist somit nicht möglich, auf die Stöchiometrie des Proteinkomplexes zu schließen, was jedoch eine wichtige Information ist, die benötigt wird, um Proteinfunktionen zu untersuchen.

Ein zweites Verfahren, welches in der Elektronenmikroskopie verwendet wird, ist die Markierung über einen natürlichen Liganden für ein Rezeptorprotein. Der Ligand ist typischerweise ein kleines Molekül, das spezifisch an einen bestimmten Rezeptor bindet. Es wird über einen relativ kurzen Linker (5 - 10 nm Länge) an ein Nanopartikel gebunden. Aufgrund der geringen Größe des Liganden und des Linkers ist es möglich, Rückschlüsse auf den Dimerisierungszustand des Proteins zu ziehen. Allerdings hat auch dieses Verfahren seine Grenzen. Die Bindung des Liganden löst oftmals einen Prozess in der Zelle aus, welcher in der Regel zu Konformationsänderungen und damit einer Veränderung des stöchiometrischen Zustands führt. Obwohl diese Methode für bestimmte Studien interessant ist, ist es in vielen anderen Fällen erwünscht, das Protein in seinem Anfangszustand (vor der Ligandenbindung) zu erfassen. Nachteilig ist bei der Verwendung von natürlichen Liganden auch, dass die Gruppe der verfügbaren Rezeptor-Proteine mit (passenden) Liganden beschränkt ist, wodurch eine große Gruppe von relevanten Proteinen nicht durch diese Methode markiert werden kann.

Ein drittes Verfahren, welches in der Elektronenmikroskopie eingesetzt wird, verwendet genetisch kodierte fluoreszierende Markierungen, die verwendet werden, um eine Metallfärbung an einer Stelle eines Proteins zu fällen, wenn diese mit Licht einer bestimmten Wellenlänge bestrahlt werden. Aber auch diese Methode ist beschränkt, da sie erfordert, dass die Zelle genetisch verändert wurde und der Fällungsprozess nur für tote und fixierte Zellen funktioniert.

WO2004020453A2 beschreibt die Bindung an DNA oder CNTs zum Aufbau von elektronischen Strukturen.

WATANABE SHIGEKI ET AL: ("Protein localization in electron micrographs using fluorescence nanoscopy", NATURE METHODS, Bd. 8, Nr. 1, Seite 80) beschreibt ein Verfahren zur Korrelierung von STED-Mikroskopie und PALM auf Gewebeproben, um Proteine zu lokalisieren und diese mit Elektronenmikroskopie zu korrelieren. Zur Ausrichtung der Aufnahmen werden fluoreszente Silika-Nanopartikel oder Goldnanopartikel verwendet. Daher besteht die Aufgabe der Erfindung darin, ein Verfahren zur Identifizierung der statistischen Verteilung der Proteinstöchiometrie mittels Elektronenmikroskopie bereitzustellen, um die oben genannten Nachteile zu beseitigen.

Das Verfahren verwendet eine spezifische Proteinmarkierung, wobei die spezifische Proteinmarkierung zwei Einheiten, eine erste Einheit und eine zweite Einheit, umfasst, wobei
- die erste Einheit ein kleines Molekül zum spezifischen Binden an ein Protein sowie mindestens ein chemisch gekoppeltes Molekül zum Anbinden an die zweite Einheit umfasst und
- die zweite Einheit ein oberflächenmodifiziertes Nanopartikel umfasst, wobei das oberflächenmodifizierte Nanopartikel eine Oberflächenbeschichtung aufweist, welche zumindest ein Molekül zum Anbinden an die erste Einheit umfasst.

Es ist hierbei vorgesehen, dass die beiden Einheiten vor ihrer Verwendung separat vorliegen. Weiterhin ist vorgesehen, dass das kleine Molekül (zum spezifischen Binden an ein Protein) der ersten Einheit aus einer Peptidsequenz, einer DNA-Sequenz oder einem kleinen chemischen Molekül besteht.

Das (kleine) Molekül zum spezifischen Binden an ein Protein wird gebildet aus einer Peptidsequenz, die aus höchstens 60 Aminosäuren besteht, einer RNA- oder DNA-Sequenz, die aus höchstens 70 Nukleotiden besteht oder einem chemischen Molekül,
dessen molekulares Gewicht höchstens 12 kDa beträgt. Die Bindungsaffinität des (kleinen) Moleküls liegt im unteren mikromolaren bis mittleren picomolaren Bereich.

Eine mögliche Verwendung der spezifischen Proteinmarkierung könnte beispielsweise die Verwendung der Markierung zur Detektion des HER2-Proteins, neuer Krebsmedikamente sowie als möglicher Kandidat für die Diagnose von Krebs liegen. HER2 ist insbesondere von Bedeutung für Brustkrebs, da es bei ca. einem Drittel aller bösartigen Brusttumoren überexprimiert ist und dies signifikant mit einer erniedrigten Überlebensdauer korreliert. Hierbei kann ein Peptid verwendet werden, welches spezifisch an HER2 binden kann und das verwandt ist mit der Domain des staphylococcal Protein A (SPA), welches ungefähr 1-13 Substitutionsmutationen aufweist.

Eine Ausgestaltung der Erfindung sieht vor, dass das Nanopartikel aus einem Material mit hoher Atomzahl (Z) besteht.

Elemente ab der IV. Periode und die Elemente der Nebengruppenelemente inkl. Lanthanoide und Actinoide werden hierbei bevorzugt.

Insbesondere ist zu der Erfindung gehörig, dass das Nanopartikel aus Gold (Au), Platin (Pt), einem anderen Edelmetall oder Cadmiumselenid (CdSe), Zinksulfid (ZnS) oder Bleiselenid (PbSe) besteht.

Beispielsweise könnte man auch Silber (Ag)-Nanopartikel verwenden.

Es ist weiterhin vorteilhaft, dass das Nanopartikel kugelförrnig, ellipsenförmig oder stäbchenförmig ist.

Ebenfalls ist im Rahmen der Erfindung vorgesehen, dass der Durchmesser des Nanopartikels im Bereich von 0,5 bis 15,0 nm liegt.

Zum Anbinden der ersten Einheit mit der zweiten Einheit ist vorteilhaft, dass die Beschichtung mindestens ein Streptavidinprotein enthält und das chemisch gekoppelte Molekül ein Biotinprotein ist.

Weitere mögliche Adhärenzmolekülverbindungen können beispielsweise Amidbindungen, Gold-Schwefelbindungen und auch van der Waals Bindungen sein.

Eine weitere Ausgestaltung der Erfindung sieht vor, dass die Nanopartikel fluoreszierende Nanopartikel sind.

Beispielsweise ist die Verwendung von etwa 10 nm großen CdSe, ZnS, InP oder PbSe-Nanopartikeln, sogenannte Quantumdots (QDs), vorstellbar. Auch Quantumdots aus anderen Materialien sind vorstellbar und zu der Erfindung gehörig.

Der Verwendung von fluoreszierenden Nanopartikeln ist vorteilhaft, wenn zur Detektion neben einem Elektronenmikroskop auch eine Fluoreszensbestimmung in einem Lichtmikroskop durchgeführt wird. Die Markierungen mit dem fluoreszierenden Nanopartikel können auch ausschließlich mittels Lichtmikroskop verwendet werden. Bei alleiniger Verwendung eines Lichtmikroskops würde bereits die Markierungseffizienz und die Markierungsspezifität verbessert werden.

Weiterhin ist vorteilhaft vorgesehen, dass die Oberflächenbeschichtung eine Tensid-Beschichtung ist. Dies ist zum Beispiel der Fall bei der Oberflächenbeschichtung der Nanopartikel mit Streptavidinmolekülen.

Eine Tensid-Beschichtung ist insbesondere vorteilhaft für das Lösungsverhalten der oberflächenmodifizierten Nanopartikel in wässrigen Lösungen.

Die Aufgabe wird durch ein Verfahren zur Identifizierung der statistischen Verteilung der Proteinstöchiometrie in Zellen unter Verwendung der spezifischen Proteinmarkierung gemäß Anspruch 1 gelöst:
Verfahren zur Identifizierung der statistischen Verteilung der Proteinstöchiometrie in Zellen umfassend die Schritte:
a) Kultivierung der Zellen in einem geeigneten Medium,
b) Inkubation der Zellen mit einer ersten Einheit, welche ein Molekül zum spezifischen Binden an ein Protein sowie mindestens ein chemisch gekoppeltes Molekül zum Anbinden an eine zweite Einheit umfasst, wobei die zweite Einheit ein oberflächenmodifiziertes Nanopartikel umfasst, welches eine Oberflächenbeschichtung aufweist, welche zumindest ein Molekül zum Anbinden an die erste Einheit umfasst, wobei das Molekül zum spezifischen Binden aus einer Peptidsequenz gebildet wird, die aus höchstens 60 Aminosäuren besteht, aus einer RNA- oder DNA-Sequenz gebildet wird, die aus höchstens 70 Nukleotiden besteht, oder ein chemisches Molekül ist, dessen molekulares Gewicht höchstens 12 kDa beträgt, in dem geeigneten Medium,
c) Waschen der Zellen,
d) Chemische oder thermische Fixierung der Zellen,
e) Inkubation der fixierten Zellen mit der zweiten Einheit, wobei sich die zweite Einheit in einem geeigneten Medium befindet,
f) Waschen der Zellen,
g) Aufnahme von elektronenmikroskopischen Bildern mittels eines Elektronenmikroskops
h) statistische Auswertung der Aufnahmen, ob die mit Nanopartikel markierten Proteine einzeln oder in einem Proteinkomplex vorliegen.

Das Verfahren zur Detektion der Position eines Proteins ist wie folgt: Die Zellen bzw. die Zelle werden/wird unter Verwendung der von qualifizierten Technikern bekannten Verfahren kultiviert. Die erste Einheit der spezifischen Proteinmarkierung, die das spezifische Bindungspeptid, DNA- oder RNA- Sequenz, oder kleines chemisches Molekül, enthält, wird mit den Zellen in einem geeigneten Medium inkubiert. Die Konzentration der spezifischen Proteinmarkierung und die Inkubationszeit werden für eine effiziente und spezifische Detektion optimiert. Typischerweise wird die spezifische Proteinmarkierung mit einer Konzentration, die zwei bis drei Größenordnungen größer ist als die Bindungsaffinität des Liganden zum Proteins von Interesse, angewandt. Die Inkubationszeit beträgt gewöhnlich einige Minuten. Danach werden die überschüssigen Moleküle der ersten Einheit der spezifischen Proteinmarkierung weggewaschen und die Zellen werden chemisch, thermisch oder physikalisch fixiert.

Eine Variation diese Methode sieht vor, dass die Zellen in einem geeigneten Medium, das Substanzen, zum Beispiel Rinderserumalbumin, enthält inkubiert werden und unspezifische Bindungen der im folgenden eingesetzten Markierungs-Einheiten blockiert werden. Die Inkubation mit dem geeigneten Medium findet vorzugsweise zwischen Schritt a) and b) statt, also bevor der Inkubation der Zellen mit den Molekülen (kleines Molekül und chemisch gekoppeltes Molekül) der ersten Einheit in dem geeigneten Medium.

Chemisch werden die Zellen beispielsweise mit Paraformaldehyd und einer niedrigen Konzentration von Glutaraldehyd fixiert, um eine Vernetzung der Proteine zu erreichen, die Zellprozesse zu stoppen und den zellulären Zustand zu erhalten. Anstelle der chemischen Fixierung ist es auch möglich, die Zellprozesse mit anderen Verfahren zu verlangsamen, beispielsweise durch das Absenken der Temperatur (thermische Fixierung).

Es ist bei dem Verfahren ebenfalls vorstellbar, dass die Zellen bereits vor der Inkubation mit dem ersten Teil der spezifischen Proteinmarkierung fixiert werden.

Nach der Inkubation der Zellen mit der ersten Einheit der spezifischen Proteinmarkierung werden die Zellen mit der zweiten Einheit der Markierung, dem oberflächenmodifizierten Nanopartikel, inkubiert. Die Bindung der Nanopartikel an die Bindungsstellen ist in der Regel nicht so effizient wie die der Peptide an das Protein. Dies ist durch die Größe und Masse der Nanopartikel begründet. Weitere Faktoren in praktischen Versuchen sind, dass die Konzentration der Nanopartikel häufig aus Kostengründen beschränkt ist und die Nanopartikel bei höheren Konzentrationen unspezifische Bindungen eingehen können. Wenn die Zellen jedoch erfindungsgemäß fixiert sind, kann die Inkubation mit der zweiten Einheit der spezifischen Proteinmarkierung in Richtung effiziente Markierung optimiert werden, beispielsweise durch die Erhöhung der Inkubationszeit auf 30 Minuten und/oder durch Zufügung von Stoffen, die für die Nanopartikel möglichen unspezifischen Bindungsstellen abdecken.

Die spezifische Proteinmarkierung des erfindungsgemäßen Verfahrens wird durch die beiden Einheiten, der ersten und der zweiten Einheit, während des Verlaufs des Experiments mit den Zellen verbunden.

Nachdem die spezifische Proteinmarkierung mit den Zellen verbunden ist, können die Zellen mittels Elektronenmikroskopie abgebildet werden. Durch die gewonnenen Lokalisationsinforrnationen der einzelnen Proteine ist es ohne weiteres möglich, festzustellen, ob Proteine in einem Proteinkomplex vorliegen oder nicht. Dabei ist es möglich, dass die Proteine als Einzelproteine, als Dimere oder in Clustern einer höheren Ordnung vorliegen. Durch die Auswertung der Elektronenmikroskopiebilder bzw. Lokalisationsanalyse von Vielen Proteinen ist es dann möglich, statistische Informationen über die stöchiometrische Verteilung der Proteine zu erhalten.

Ein Vorteil der oben beschriebenen Zweischritt-Markierung ist das Erreichen einer optimalen Markierungseffizienz und Spezifität.

Ein weiterer wichtiger Vorteil ist, dass durch das erfindungsgemäße Verfahren ermöglicht wird, die Markierung mit einem Nanopartikel, das mehrere Adhärenzmoleküle aufweist, durchzuführen, ohne Clustering-Artefakte zu erzeugen.

Dies wird nachfolgend durch ein Gegenbeispiel erläutert: Markierungen, die aus kleinen Peptiden erzeugt sind, koppeln mit oberflächenmodifizierten Nanopartikeln, die in ihrer oberflächenmodifizierten Beschichtung mehrere Bindungsmoleküle aufweisen. Diese werden mit den Zellen in einem Einschritt-Verfahren inkubiert. Wenn eine Markierung mit mehreren Bindungsstellen mit lebenden Zellen inkubiert wird, induziert die Markierung Clustering, denn nach der anfänglichen Bindung eines Nanopartikels an ein Protein koppeln benachbarte Proteine durch Diffusionsprozesse ebenfalls an die verfügbaren Bindungsstellen des Nanopartikels, so dass ein Nanoteilchen eine Anzahl von Proteinen konzentriert. Diese Clusterbildung verhindert eine zuverlässige Untersuchung der statistischen Verteilung der Stöchiometrie der Zielproteinspezies.

Bei der Verwendung der erfindungsgemäßen Markierung und des zweistufigen Verfahrens bindet das Peptid zuerst an das Zielprotein. Die zweite Einheit der proteinspeziflschen Markierung, die das oberflächenmodifizierte Nanopartikel aufweist, wird erst nach dem Verfahrensschritt der Fixierung aufgebracht, so dass Clustering nicht stattfinden kann. Somit kann die erfindungsgemäße Markierung verwendet werden, um die Stöchiometrie zu detektieren.

Ein weiterer Vorteil der aufeinanderfolgend aufgebrachten Einheiten der proteinspezifischen Markierung ist, dass eine große Flexibilität in den Experimenten erreicht wird.

Die gestellte Aufgabe wird ebenfalls durch ein bevorzugtes Verfahren gemäß dem abhängigen Anspruch 2 gelöst:
Verfahren zur Identifizierung der statistischen Verteilung der Proteinstöchiometrie in Zellen nach Anspruch 1, wobei in Schritt b) die Zellen mit mindestens zwei unterschiedlichen ersten Einheiten in dem geeigneten Medium inkubiert werden, und in Schritt e) die Zellen mit mindestens zwei unterschiedlichen zweiten Einheiten inkubiert werden, wobei sich die zweiten Einheiten in einem geeigneten Medium befinden und jede der zweiten Einheiten zumindest ein Molekül umfasst, welches an eine der ersten Einheiten adhäriert.

Es ist erfindungsgemäß vorgesehen, dass bei dieser Lösung der Aufgabe zwei oder mehr unterschiedliche Proteine jeweils spezifisch mit verschiedenen Markierungen markiert sind. Ebenfalls ist es zu der Erfindung gehörig, dass jede der zweiten Einheiten mindestens ein Molekül umfasst, das sich spezifisch mit der ersten Einheit verbindet.

In der Krebsdiagnostik sind verschiedene kleine Peptid-Sequenzen, zusätzlich zu derjenigen, die das HER2 bindet, bekannt. Beispielsweise sind Peptid-Sequenzen bekannt, die eine Bindung mit EGFR oder HER3 eingehen. Alle drei Proteine weisen eine hohe Relevanz für die Krebsdiagnose und Krebstherapie auf.

Die zweite Einheit der proteinspezifischen Markierungen kann mittels der verwendeten Art des Nanopartikels in elektronenmikroskopischen Bildern identifiziert werden. Die Nanopartikel können beispielsweise eine Größe von 1,4 nm, 5 nm oder 10 nm aufweisen, kugelförmig, ellipsenförmig oder stabförmig sein oder aus unterschiedlichen Materialien, wie beispielsweise Gold, Platin, CdSe/ZnS oder PbSe hergestellt sein. Durch die Unterschiede in den Nanopartikeln (Größe, Form, Material, etc.) in den unterschiedlichen spezifischen Proteinmarkierungen können die Zielproteine identifiziert und lokalisiert werden. Zusätzlich steht bei Verwendung von fluoreszierenden Nanopartikeln, zum Beispiel Quantenpunkten (Quantumdots, QDs), eine lichtoptische Signatur zur Verfügung.

Durch eine Kombination von spezifischen Proteinmarkierungen wird ermöglicht, die Stöchiometrie von Proteinkomplexen, die aus verschiedenen Arten von Proteinen bestehen, zu erkennen und auszuwerten. Hierdurch kann eine wichtige Frage der biologischen Forschung zu den Mechanismen, die bei der Entwicklung von Brustkrebs eine Rolle spielt, geklärt werden, nämlich in welcher Konzentration die EGFR-HER2-hetero-Dimerbildung und EGFR-HER2-hetero-Dimerhemmung erfolgt.

Die Fixierung in Schritt d) kann chemisch oder thermisch erfolgen. Chemisch werden die Zellen beispielsweise mit Paraformaldehyd und einer niedrigen Konzentration von Glutaraldehyd fixiert, um eine Vernetzung der Proteine zu erreichen, die Zellprozesse zu stoppen und den zellulären Zustand zu erhalten. Anstelle der chemischen Fixierung ist es auch möglich, die Zellprozesse mit anderen Verfahren zu verlangsamen, beispielsweise durch das Absenken der Temperatur (thermische Fixierung).

Eine Variation dieser Methode ist die sequentielle Verarbeitung der unterschiedlichen Einheiten, d.h. Inkubation mit der ersten Einheit von einem Label, Waschen, Inkubation mit der ersten Einheit von einer zweiten Markierung, Fixierung, usw. Es ist ebenfalls vorstellbar, dass die Fixierung nach der Inkubation mit der ersten Einheit erfolgt.

Beispielhaft kann der EGFR-Dimer mit zwei unterschiedlichen Markierungen studiert werden, wobei eine Gold-Nanopartikel-Markierung, welche an dem EGF-Liganden befestigt ist, und als weitere Markierung ein kleines Bindungspeptid, das an einen Quantumdot (QD) gebunden ist, verwendet wird.

Zuerst erfolgt die Inkubation der Zellen mit EGF und dem Bindungspeptid. Danach werden die Zellen fixiert. Nanopartikel werden dann an die Liganden gekoppelt, zum Beispiel durch die Verwendung von EGF-Biotion und Nanopartikel-Streptavidin. Beispielsweise kann das Nanopartikel auch vor der Inkubation an den EGF gekoppelt werden. Nun wird die aus zwei Einheiten bestehende spezifische Proteinmarkierung als zweite Markierung eingesetzt, wobei die zwei Einheiten nacheinander eingesetzt werden, jedoch wird dabei als Nanopartikel ein anderer als an den Liganden gekoppelten Nanopartikel eingesetzt, damit Liganden und Protein separat zu detektieren sind.

Zum Beispiel wird an dem Ligand EGF ein QD gekoppelt und mittels der aus zwei Einheiten bestehenden spezifischen Proteinmarkierung ein Gold-Nanopartikel an das EGFR-Protein gekoppelt. Da die Bindung des Liganden das EGFR-Protein aktiviert, können mittels der QDs die Rezeptoren, welche aktiviert wurden, lokalisiert werden. Die Gold-Nanopartikel lokalisieren hingegen alle EGFRs.

Es wird daher ermöglicht, die wichtige biologische Frage zu beantworten, ob alle Dimere aktivierte EGFRs beinhalten oder ob - und wenn ja wie viele -EGFRs bereits in inaktiver Form als Dimere vorhanden sind.

In weitereren Variationen diese Methode können auch andere Reihenfolgen der obengenannten Arbeitsschritte verwendet werden. Zum Beispiel wird zuerst mit der aus zwei Einheiten bestehenden spezifischen Proteinmarkierung markiert, gefolgt von der Markierung mittels des natürlichen Liganden. Es ist jedoch auch im Rahmen der Erfindung vorgesehen, dass natürliche Liganden und die erste Einheit der spezifischen Proteinmarkierung gleichzeitig eingesetzt werden.

Es ist weiterhin zu der Erfindung gehörig, dass Bilder von Zellen, in denen verschiedene Proteine mit den aus zwei Einheiten bestehenden spezifischen Proteinmarkierungen markiert sind, aufgenommen werden.

Das zum Einsatz gebrachte Elektronenmikroskop kann ein Transmissionselektronenmikroskop, ein Raster-Transmissionselektronenmikroskop oder ein Raster-Elektronenmikroskop sein.

Es ist weiterhin zu der Erfindung gehörig, dass die Aufnahmen von Elektronenmikroskopbildern mittels einer Vorrichtung für die Raster-Transmissionselektronenmikroskopie (STEM) durchgeführt werden, wobei ein STEM-Detektor mit Empfindlichkeit für Atomzahl, sogenannter Z-Kontrast, eingesetzt wird. Der STEM Detektor sorgt dafür, dass die Marker bzw. Markierungen mit deutlich höherem Kontrast als das umliegende Zellmaterial in den Bildern sichtbar sind.

Es ist weiterhin zu der Erfindung gehörig, dass die Markierungen manuell oder anhand eines automatischen Verfahrens in den Bildern detektiert werden können, sodass Informationen über die Position und des Typs der markierten Proteine erhalten werden. Diese Informationen können für mehrere verschiedene Experimente ausgewertet und daraufhin miteinander verglichen werden. Beispielsweise kann somit identifiziert werden, ob sich das Vorkommen der EGFR-Dimere nach Zugabe eines Krebsmedikaments verändert.

Es ist weiterhin zu der Erfindung gehörig, dass lichtmikroskopische Bilder der betreffenden Zellen erstellt werden.

Diese Information kann in Bezug zu den Markierungspositionen und Typen gesetzt werden, d.h. korreliert werden, wodurch zum Beispiel untersucht werden kann, in welchen Bereichen ein bestimmtes Markierungsmuster vorkommt.

Es ist weiterhin zu der Erfindung gehörig, dass für die aus zwei Einheiten bestehenden spezifischen Proteinmarkierungen fluoreszenzmikroskopische Bilder mit elektronenmikroskopischen Bildern kombiniert werden. Zum Beispiel können die genauen Positionen von mit QD markierten Proteinen mit dem Vorkommen der Fluoreszenz der QD Typ entsprechenden Wellenlänge in fluoreszenzmikroskopische Bildern verglichen werden. Aus diesen Informationen kann in Erfahrung gebracht werden, in welchen Zellen oder zellulären Regionen ein bestimmtes Protein vorliegt. Weiterhin kann damit untersucht werden ob es lokale Variationen in der Dichte/Häufigkeit der Dimere, sowohl zwischen verschiedenen Zellen wie auch innerhalb einer einzelnen Zelle, gibt.

Zudem kann mittels Licht- und Fluoreszenzmikroskopie eine lebende Zelle zeitaufgelöst untersucht werden. Zelluläre Prozesse können daher untersucht werden. Sobald sich ein bestimmter Prozess in einem bestimmten Stadium befindet, kann eine bestimmte Phase der Markierung oder Fixierung initiiert werden, damit die Markierungsinformation zeitlich mit einem zellulären Prozess korreliert ist. Auch kann damit der genaue Zeitpunkt der Markierung nach Zugabe eines Wirkstoffes, z.B. eines krebshemmenden Medikaments, bestimmt werden.

Es ist weiterhin zu der Erfindung gehörig, dass die Aufnahmen von Elektronenmikroskopbildern mittels einer Vorrichtung für die korrelative Raster-Transmissionselektronenmikroskopie (STEM) und Lichtmikroskopie durchgeführt werden, wobei ein STEM-Detektor mit einer lichtoptischen Linse kombiniert ist.

Diese Detektionsvorrichtung kombiniert die effiziente Detektion mittels STEM-Mikroskopie von Materialien mit hoher Atomzahl, beispielsweise spezifischen Nanopartikel-Markern in einer Probe in einer Flüssigkeit, wie einer Zelle, mit gleichzeitiger Lichtmikroskopie, beispielsweise über Fluoreszenzkontrast an fluoreszierenden Proteinmarkern in Zellen oder über Streukontrast des Zellmaterials. Durch die Verwendung einer solchen Vorrichtung werden eine höchst effiziente Detektion von Materialien mit hoher Atomzahl in der Probe mit größtmöglicher Auflösung und eine vollständig zeitkorrelative Lichtmikroskopie ermöglicht.

Eine bevorzugte Ausbildung für die Vorrichtung besteht darin, dass der STEM-Detektor in eine lichtoptische Linse integriert ist.

In diesem Zusammenhang ist es vorteilhaft, dass der STEM-Detektor in einem Hohlraum in der lichtoptischen Linse angeordnet ist.

Die Vorrichtung kann auch derart ausgestaltet sein, dass der Hohlraum probenseitig eine Öffnung mit geringem Durchmesser aufweist, an die sich ein konisch ausgebildeter Elektronendriftraum anschließt, an dessen unteren Ende der STEM-Detektor angeordnet ist.

Unten bezieht sich hierbei auf eine Elektronenstrahlrichtung von oben nach unten. Beliebige Strahlenrichtungen sind möglich, abhängig von der Anordnung der Vorrichtung. Dies bedeutet, dass auf der der Probenhalterung zugewandten Seite der Linse die Öffnung angeordnet ist, an die sich der konisch nach unten erweiternde Elektronendriftraum anschließt, an dessen unteren Ende der STEM-Detektor angeordnet ist.

Das Signal des STEM-Detektors kann auf der Seite der Linse nach außen ausführbar sein.

Die Vorrichtung kann auf der der Probe zugewandten Seite der lichtoptischen Linse eine Probenhalterung aufweisen, wobei die Probenhalterung als dünne elektronendurchlässige Membran ausgebildet sein kann, welche elektronendurchlässig ausgebildet ist.

Für die Aufnahmen von Elektronenmikroskopbildern, die mittels einer Vorrichtung für die korrelative Raster-Transmissionselektronenmikroskopie (STEM) und Lichtmikroskopie durchgeführt werden, sollte der Raum um die Probenhalterung und der Elektronendriftraum die Eigenschaft besitzen, ein Vakuum einzustellen.

Für die Aufnahmen von Elektronenmikroskopbildern, die mittels einer Vorrichtung für die korrelative Raster-Transmissionselektronenmikroskopie und Lichtmikroskopie durchgeführt werden, kann weiterhin vorgesehen sein, dass eine Elektronenstrahlquelle auf der dem STEM-Detektor gegenüberliegenden Seite der der Probenhalterung angeordnet ist.

Die Detektionsvorrichtung kann auch eine Linse aufweisen, wobei eine Lichtquelle und lichtoptische Detektionsmittel mit der Linse verbunden sind.

Die Linse mit integriertem STEM-Detektor kann in unterschiedliche Elektronenmikroskope eingebaut werden, z.B. ein ESEM mit einer typischen Elektronenenergie von 30 keV oder ein hochauflösendes STEM mit einer typischen Elektronenenergie von 200 keV.

Es ist vorteilhaft, wenn die Detektionsvorrichtung für die korrelative Raster-Transmissionselektronenmikroskopie und Lichtmikroskopie ein oder mehrere andere lichtoptische Strahlengänge zur Detektion oder zur Beleuchtung aufweist.

Für die Aufnahmen von Elektronenmikroskopbildern, die mittels einer Vorrichtung für die korrelative Raster-Transmissionselektronenmikroskopie und Lichtmikroskopie durchgeführt werden, ist es ebenfalls möglich, dass die Lichtquelle seitlich des STEM-Detektors angeordnet ist, der Lichtstrahl sich im Fokus mit dem Elektronenstahl überlappt und der lichtoptische Detektionsweg sich mit dem Beleuchtungsstrahl überlappt.

Man könnte jedoch auch für die Aufnahmen von Elektronenmikroskopbildern, die mittels einer Vorrichtung für die korrelative Raster-Transmissionselektronenmikroskopie und Lichtmikroskopie durchgeführt werden, eine Vorrichtung verwenden, bei der der STEM-Detektor zwischen der optischen Linse und der Probe angeordnet ist. In gleicher Weise ist es möglich, dass der STEM-Detektor in den Bereich zwischen der optischen Linse und der Probe verfahrbar ist.

Im Folgenden wird der Stand der Technik sowie ein Ausführungsbeispiel der Erfindung anhand von Zeichnungen erläutert.

Es zeigen
- Fig. 1: eine teilschematische Darstellung für den Abstand von Proteininteraktionen eines Dimers (hier EGFR),
- Fig. 2: eine teilschematische Darstellung der Verwendung eines Gold-Nanopartikels, das über einen Linker mit einem Zielprotein (EGFR-Dimer) verbunden ist,
- Fig. 3: die erfindungsgemäße spezifische Proteinmarkierung in teilschematische Darstellung, die mit einem EGFR-Dimer verbunden ist.

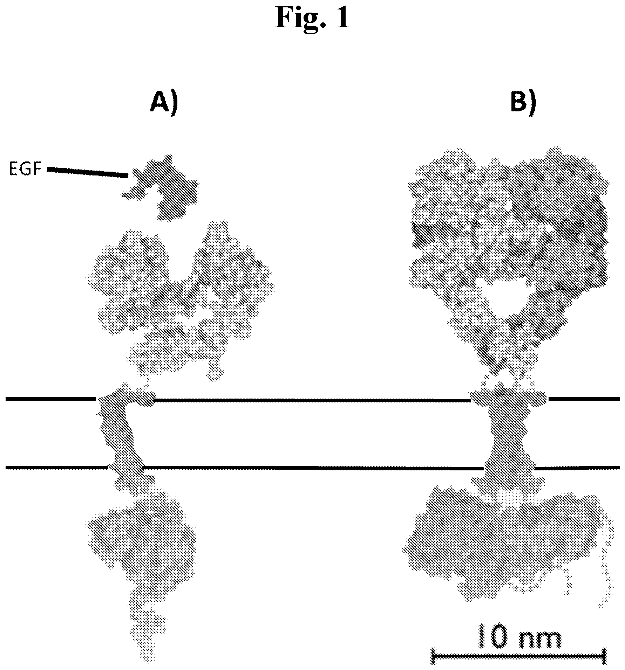

In den Fig. 1 A) und 1 B) ist ein epidermaler Wachstumsfaktor (epidermal growth factor, EGF)-Rezeptor (EGFR)-Dimer in der Plasmamembran dargestellt. Fig. 1 A) zeigt dabei einen nicht aktivierten EGFR sowie einen ungebundenen epidermalen Wachstumsfaktor (EGF). Fig. 1 B) zeigt einen aktiven EGRF-Dimer mit gebundenem EGF-Liganden. Beispielsweise ist der Längenmaßstab, bei dem Proteininteraktionen stattfinden für die Dimerbildung des epidermalen Wachstumsfaktors-Rezeptors (EGFR) in Fig. 1 B) gezeigt. Der epidermale Wachstumsfaktorrezeptor spielt eine kritische Rolle in der Pathogenese und Entwicklung von vielen verschiedenen Krebsarten.

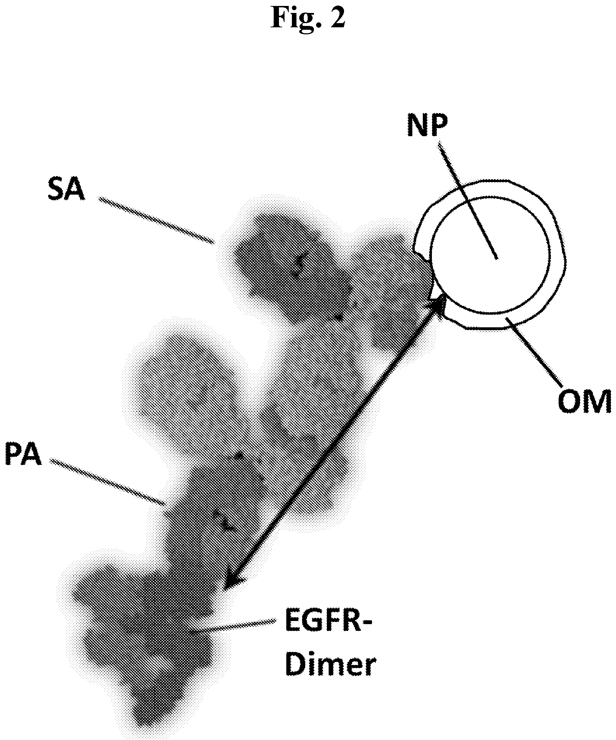

Fig. 2 beschreibt einen EGFR-Dimer, der über einen Antikörper-Linker, bestehend aus einem primären Antikörper (PA) und einem sekundären Antikörper (SA) mit einer Gold-Nanopartikel (NP) verbunden ist. Der primäre Antikörper (PA) bindet spezifisch an das Protein (EGFR) und der sekundäre Antikörper (SA) an ein Gold-Nanopartikel (NP), welches als Oberflächenmodifizierung (OM) mindestens ein chemisches Molekül für die Bindung mit dem Antikörper aufweist. Der Abstand (Pfeil) zwischen dem Nanopartikel (NP) und dem Protein (EGFR-Dimer) kann bis zu 30 nm betragen.

Viele Studien verwenden diese Immunogoldmarkierungen, wobei jedoch die Nanopartikelmarkierungen mit den Zielproteinen über einen ziemlich langen Linker (siehe Fig. 2, SA + PA) befestigt werden. Der Linker ist flexibel und hat eine typische Länge von 30 nm. Als direkte Folge der Länge des Linkers kann die genaue Lage des Proteins (EGFR), bzw. beider Proteinkomponenten im Fall des EGFR-Dimers, nicht mit einer besseren Genauigkeit als 30 nm, bzw. 60 nm für den Dimer, bestimmt werden, was diese Anwendung stark einschränkt. Die Immunogoldmarkierung liefert Informationen über die Anwesenheit einer bestimmten Proteinspezies, zum Beispiel in einer Organelle, aber kann keine Informationen über den genauen Ort des Proteins mit einer besseren Genauigkeit als 30 nm zeigen. Im Extremfall würden sogar zwei benachbarte Nanopartikel (NP) zwei EGFRs darstellen, welche maximal 60 nm voneinander beabstandet sind. Dieser Abstand wäre wesentlich größer als die Größe eines Protein-Komplexes. Es ist also nicht möglich, aus den Daten zu schließen, ob zwei benachbarte beobachtete Markierungen ein Dimer oder etwa zwei Proteine, die einen beträchtlichen Abstand voneinander haben, darstellen. Kürzere Linker-Antikörper, um einen Faktor zwei kleiner, existieren, aber werden selten verwendet, da der Abstand immer noch zu groß für den Nachweis der Stöchiometrie ist.

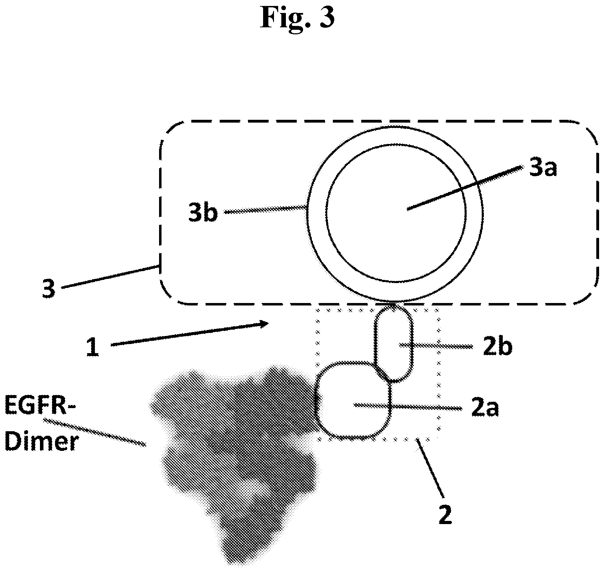

Fig. 3 beschreibt die erfindungsgemäße spezifische Proteinmarkierung (1). Hierbei wird beispielshaft ein EGFR-Dimer dargestellt, an dem die erfindungsgemäße spezifische Proteinmarkierung (1) bindet, um den Dimer mittels Elektronenmikroskopie nachzuweisen. Die Peptidsequenz (2a) ist mit einem kleinen Molekül (2b), welches sich mit der Oberflächenbeschichtung (3b) eines oberflächenmodifizierten Nanopartikels (3a) verbindet. Die spezifische Proteinmarkierung (1) besteht aus zwei Teilen: Das Bindungspeptid (2a) und das kleine Molekül (2b) bilden einen ersten Teil (gepunkteter Bereich 2) und das oberflächenmodifizierte Nanopartikel (3a) mit seiner Oberflächenbeschichtung (3b) bildet den zweiten Teil (gestrichelter Bereich 3).

## Patentansprüche

1. Verfahren zur Identifizierung der statistischen Verteilung der Proteinstöchiometrie in Zellen umfassend die Schritte:
a) Kultivierung der Zellen in einem geeigneten Medium,
b) Inkubation der Zellen mit einer ersten Einheit (2), welche ein Molekül (2a) zum spezifischen Binden an ein Protein sowie mindestens ein chemisch gekoppeltes Molekül (2b) zum Anbinden an eine zweite Einheit (3) umfasst, wobei die zweite Einheit (3) ein oberflächenmodifiziertes Nanopartikel (3a) umfasst, welches eine Oberflächenbeschichtung (3b) aufweist, welche zumindest ein Molekül zum Anbinden an die erste Einheit (2) umfasst, wobei das Molekül (2a) zum spezifischen Binden aus einer Peptidsequenz gebildet wird, die aus höchstens 60 Aminosäuren besteht, aus einer RNA- oder DNA-Sequenz gebildet wird, die aus höchstens 70 Nukleotiden besteht, oder ein chemisches Molekül ist, dessen molekulares Gewicht höchstens 12 kDa beträgt, in dem geeigneten Medium,
c) Waschen der Zellen,
d) Chemische oder thermische Fixierung der Zellen,
e) Inkubation der fixierten Zellen mit der zweiten Einheit (3), wobei sich die zweite Einheit in einem geeigneten Medium befindet,
f) Waschen der Zellen,
g) Aufnahme von elektronenmikroskopischen Bildern mittels eines Elektronenmikroskops,
h) statistische Auswertung der Aufnahmen, ob die mit Nano-partikel (3a) markierten Proteine einzeln oder in einem Proteinkomplex vorliegen.

2. Verfahren zur Identifizierung der statistischen Verteilung der Proteinstöchiometrie in Zellen nach Anspruch 1, wobei in Schritt b) die Zellen mit mindestens zwei unterschiedlichen ersten Einheiten (2) in dem geeigneten Medium inkubiert werden, und in Schritt e) die Zellen mit mindestens zwei unterschiedlichen zweiten Einheiten (3) inkubiert werden, wobei sich die zweiten Einheiten (3) in einem geeigneten Medium befinden und jede der zweiten Einheiten (3) zumindest ein Molekül umfasst, welches an eine der ersten Einheiten (2) adhäriert.

3. Verfahren gemäß einem der Ansprüche 1 oder 2, **dadurch gekennzeichnet, dass** die Zellen vor der Inkubation mit der ersten Einheit (2) in einem geeigneten Medium inkubiert werden, wobei das geeignete Medium Substanzen enthält, welche die unspezifische Bindung der eingesetzten zweiten Einheit (3) blockiert.

4. Verfahren gemäß einem der Ansprüche 1 bis 3, **dadurch gekennzeichnet, dass** das Elektronenmikroskop einen STEM-Detektor umfasst.

5. Verfahren gemäß einem der Ansprüche 1 bis 4, **dadurch gekennzeichnet, dass** ein Lichtmikroskop bzw. ein Fluoreszenzmikroskop für die Aufnahme von lichtmikroskopischen Bildern bzw. Fluoreszenzbildern verwendet wird.

6. Verfahren gemäß einem der Ansprüche 1 bis 5, **dadurch gekennzeichnet, dass** während des Verfahrens mindestens eine zeitaufgelöste Serie von lichtmikroskopischen und/oder fluoreszenzmikroskopischen Bildern aufgenommen wird, wodurch mindestens ein Zeitpunkt von mindestens einem der Schritte des Verfahrens mit der lichtmikroskopischen und/oder fluoreszenzmikroskopischen Information zeitlich korreliert werden kann.

7. Verfahren gemäß einem der Ansprüche 1 bis 6, **dadurch gekennzeichnet, dass** für die Aufnahme von Elektronenmikroskopbildern eine Vorrichtung für die korrelative Raster-Transmissionselektronenmikroskopie (STEM) und Lichtmikroskopie verwendet wird, wobei ein STEM-Detektor mit einer lichtoptischen Linse kombiniert ist.

8. Verwendung einer spezifischen Proteinmarkierung (1) zur Identifizierung der statistischen Verteilung der Proteinstöchiometrie in Zellen umfassend zwei Einheiten (2, 3), eine erste Einheit (2) und eine zweite Einheit (3), wobei
- die erste Einheit (2) ein Molekül (2a) zum spezifischen Binden an ein Protein sowie mindestens ein chemisch gekoppeltes Molekül (2b) zum Anbinden an die zweite Einheit (3) umfasst und
- die zweite Einheit (3) ein oberflächenmodifiziertes Nanopartikel (3a) umfasst, wobei das Nanopartikel (3a) eine Oberflächenbeschichtung (3b) aufweist, welche zumindest ein Molekül zum Anbinden an die erste Einheit (2) umfasst, wobei das Molekül (2a) zum spezifischen Binden aus einer Peptidsequenz gebildet wird, die aus höchstens 60 Aminosäuren besteht, aus einer RNA- oder DNA-Sequenz gebildet wird, die aus höchstens 70 Nukleotiden besteht, oder ein chemisches Molekül ist, dessen molekulares Gewicht höchstens 12 kDa beträgt, in einem Verfahren nach einem der Ansprüche 1 bis 7.

## Claims

1. Method for identifying the statistical distribution of protein stoichiometry in cells, comprising the steps of:
a) culturing the cells in a suitable medium,
b) incubating the cells with a first unit (2) which comprises a molecule (2a) for specific binding to a protein as well as at least one chemically coupled molecule (2b) for attachment to a second unit (3), wherein the second unit (3) comprises a surface-modified nanoparticle (3a) having a surface coating (3b) comprising at least one molecule for attachment to the first unit (2), wherein the molecule (2a) for specific binding is formed from a peptide sequence consisting of not more than 60 amino acids, is formed from an RNA or DNA sequence consisting of not more than 70 nucleotides, or is a chemical molecule, the molecular weight of which is not more than 12 kDa, in the suitable medium,
c) washing the cells,
d) chemically or thermally fixing the cells,
e) incubating the fixed cells with the second unit (3), wherein the second unit is situated in a suitable medium,
f) washing the cells,
g) capturing electron-microscope images by means of an electron microscope,
h) statistical evaluation of the images as to whether the proteins labelled with nanoparticles (3a) are present individually or in a protein complex.

2. Method for identifying the statistical distribution of protein stoichiometry in cells according to Claim 1, wherein, in step b), the cells are incubated with at least two different first units (2) in the suitable medium and, in step e), the cells are incubated with at least two different second units (3), wherein the second units (3) are situated in a suitable medium and each of the second units (3) comprises at least one molecule which adheres to one of the first units (2).

3. Method according to either of Claims 1 and 2, **characterized in that** the cells are incubated in a suitable medium before the incubation with the first unit (2), wherein the suitable medium contains substances which block the non-specific binding of the second unit (3) used.

4. Method according to any of Claims 1 to 3, **characterized in that** the electron microscope comprises a STEM detector.

5. Method according to any of Claims 1 to 4, **characterized in that** a light microscope or a fluorescence microscope is used for the capture of light-microscope images or fluorescence images.

6. Method according to any of Claims 1 to 5, **characterized in that** at least one time-resolved series of light-microscope and/or fluorescence-microscope images is captured during the method, the result being that at least one time point of at least one of the steps of the method can be temporally correlated with the item of light-microscope and/or fluorescence-microscope information.

7. Method according to any of Claims 1 to 6, **characterized in that** a device for correlative scanning transmission electron microscopy (STEM) and light microscopy is used for the capture of electron-microscope images, wherein a STEM detector is combined with a light-optical lens.

8. Use of a specific protein label (1) for identifying the statistical distribution of protein stoichiometry in cells comprising two units (2, 3), a first unit (2) and a second unit (3), wherein
- the first unit (2) comprises a molecule (2a) for specific binding to a protein as well as at least one chemically coupled molecule (2b) for attachment to the second unit (3) and
- the second unit (3) comprises a surface-modified nanoparticle (3a), wherein the nanoparticle (3a) has a surface coating (3b) comprising at least one molecule for attachment to the first unit (2), wherein the molecule (2a) for specific binding is formed from a peptide sequence consisting of not more than 60 amino acids, is formed from an RNA or DNA sequence consisting of not more than 70 nucleotides, or is a chemical molecule, the molecular weight of which is not more than 12 kDa, in a method according to any of Claims 1 to 7.

## Revendications

1. Procédé d'identification de la répartition statistique de la stoechiométrie des protéines dans des cellules, comprenant les étapes suivantes :
a) culture des cellules dans un milieu approprié,
b) incubation des cellules avec une première unité (2), laquelle comporte une molécule (2a) destinée à se lier spécifiquement à une protéine ainsi qu'au moins une molécule chimiquement couplée (2b) destinée à se lier à une deuxième unité (3), la deuxième unité (3) comprenant une nanoparticule (3a) à modification de surface, laquelle présente un revêtement de surface (3b) qui comporte au moins une molécule destinée à se lier à la première unité (2), la molécule (2a) destinée à se lier spécifiquement étant formée à partir d'une séquence peptidique qui se compose au maximum de 60 acides aminés, étant formée à partir d'une séquence d'ARN ou d'ADN qui se compose au maximum de 70 nucléotides, ou étant une molécule chimique dont le poids moléculaire est au maximum de 12 kDa, dans le milieu approprié,
c) lavage des cellules,
d) fixation chimique ou thermique des cellules,
e) incubation des cellules fixées avec la deuxième unité (3), la deuxième unité se trouvant dans un milieu approprié,
f) lavage des cellules,
g) enregistrement d'images de microscopie électronique au moyen d'un microscope électronique,
h) interprétation statistique des enregistrements en vue de déterminer si les protéines marquées avec les nanoparticules (3a) sont présentes individuellement ou dans un complexe protéiné.

2. Procédé d'identification de la répartition statistique de la stoechiométrie des protéines dans des cellules selon la revendication 1, selon lequel, dans l'étape b), les cellules sont incubées dans le milieu approprié avec au moins deux premières unités (2) différentes et, dans l'étape e), les cellules sont incubées avec au moins deux deuxièmes unités (3) différentes, les deuxièmes unités (3) se trouvant dans un milieu approprié et chacune des deuxièmes unités (3) comportant au moins une molécule qui adhère à l'une des premières unités (2).

3. Procédé selon l'une des revendications 1 ou 2, **caractérisé en ce que** les cellules sont incubées dans un milieu approprié avant l'incubation avec la première unité (2), le milieu approprié contenant des substances qui bloquent la liaison non spécifique de la deuxième unité (3) utilisée.

4. Procédé selon l'une des revendications 1 à 3, **caractérisé en ce que** le microscope électronique comprend un détecteur STEM.

5. Procédé selon l'une des revendications 1 à 4, **caractérisé en ce qu'**un microscope optique ou un microscope à fluorescence est utilisé pour l'enregistrement d'images de microscopie optique ou d'images de fluorescence.

6. Procédé selon l'une des revendications 1 à 5, **caractérisé en ce que** pendant le procédé, au moins une série avec résolution temporelle d'images de microscopie optique et/ou d'images de microscopie à fluorescence est enregistrée, ce par quoi au moins un instant d'au moins l'une des étapes du procédé peut être corrélé dans le temps avec l'information de microscopie optique et/ou de microscopie à fluorescence.

7. Procédé selon l'une des revendications 1 à 6, **caractérisé en ce que** l'enregistrement d'images de microscopie électronique est effectué en utilisant un dispositif pour la microscopie électronique en transmission à balayage (STEM) corrélative et la microscopie optique, un détecteur STEM étant combiné avec une lentille optique.

8. Utilisation, dans un procédé selon l'une des revendications 1 à 7, d'un marquage de protéines spécifique (1) pour l'identification de la répartition statistique de la stoechiométrie des protéines dans des cellules comprenant deux unités (2, 3), une première unité (2) et une deuxième unité (3),
- la première unité (2) comportant une molécule (2a) destinée à se lier spécifiquement à une protéine ainsi qu'au moins une molécule chimiquement couplée (2b) destinée à se lier à la deuxième unité (3), et
- la deuxième unité (3) comprenant une nanoparticule (3a) à modification de surface, la nanoparticule (3a) présentant un revêtement de surface (3b) qui comporte au moins une molécule destinée à se lier à la première unité (2), la molécule (2a) destinée à se lier spécifiquement étant formée à partir d'une séquence peptidique qui se compose au maximum de 60 acides aminés, étant formée à partir d'une séquence d'ARN ou d'ADN qui se compose au maximum de 70 nucléotides, ou étant une molécule chimique dont le poids moléculaire est au maximum de 12 kDa.
